# EUROPÄISCHE PATENTSCHRIFT

(11) **EP 1 002 571 B1**
(45) Veröffentlichungstag und Bekanntmachung des Hinweises auf die Patenterteilung: **07.01.2004**
(21) Anmeldenummer: 99122144.1
(22) Anmeldetag: 05.11.1999
(51) Int. Cl.: B01J 8/02, B01J 12/00, C01B 3/16, C07C 5/09, C07C 5/03, C07C 1/04, C07C 29/152, C07D 301/02, C01B 17/04, C01B 17/80, C01C 1/04, C10G 2/00, C01B 17/02

(54) **Reaktor zur Durchführung katalytischer Reaktionen mit starker Wärmetönung**
Reactor for highly exothermic reactions
Réacteur pour réaliser des réactions fortement exothermiques

(30) Priorität: 06.11.1998 DE 19851109
(43) Veröffentlichungstag der Anmeldung: 24.05.2000
(73) Patentinhaber: Heisel, Michael Dr., 82049 Pullach (DE)
(72) Erfinder: Heisel, Michael Dr., 82049 Pullach (DE)
(74) Vertreter: Best, Michael, Dr.

(56) Entgegenhaltungen:
- WO-A-98/30856
- US-A- 1 850 398
- US-A- 3 127 247
- US-A- 4 544 544

## Beschreibung

Die Erfindung betrifft einen Reaktor zur Durchführung von Reaktionen mit starker Wärmetönung mit Katalysatorpartikeln zwischen gekühlten Trennwänden in mindestens einem Reaktorbehälter.

Aus der Zeitschrift Hydrocarbon Processing, March 1997, Seite 134, ist ein solcher Reaktor bekannt. Es handelt sich um einen Röhrenreaktor mit Katalysatorpartikeln in den Rohren. Die Rohre werden auf der Mantelseite des Reaktors mit siedendem Wasser oder anderen geeigenten Wärmeträgern gekühlt.

Die Aufteilung des Reaktionsraumes und der Katalysatorpartikel auf mehrere Rohre stellt sicher, daß im Falle einer Betriebsstörung eine sich selbst beschleunigende Reaktion, hervorgerufen durch örtliche Überhitzung, sich auf ein Reaktionsrohr beschränkt und nicht den ganzen Reaktor erfaßt. Diese Reaktorkonstruktion hat sich bewährt, weist jedoch auch mehrere Nachteile auf.
- Der Reaktormantel muß auf den Kühlmitteldruck ausgelegt sein, was in der Praxis oft hohe Drücke sind. Dadurch ist der Mantel sehr dick und damit teuer, schwer zu transportieren und Baustellenmontage ist ausgeschlossen.
- Die Rohrböden sind bei großem Durchmesser sehr dick und damit teuer und durch Wärmespannungen gefährdet.
- Die vielen Reaktionsrohre sind nur mit großem Aufwand in die dicken Rohrböden einzuschweißen.
- Die vielen Reaktionsrohre sind nur mit großem Aufwand zu befüllen. Insbesondere ist auf gleichmäßige Befüllung mit gleichem Druckverlust in den verschiedenen Rohren zu achten, damit nicht ein wegen hohem Druckabfall zu wenig beaufschlagtes Reaktionsrohr überhitzt wird.
- Wegen des hohen Gewichts wird für den Reaktor meist C-Stahl verwendet, obwohl Rost damit unvermeidlich ist. Rost wirkt aber für viele Reaktionen als ein Katalysatorgift. Bei Katalysatorentleerung muß deshalb der Reaktor sandgestrahlt werden, was bei der großen Zahl von Reaktionsrohren ein erheblicher Aufwand ist.
- Nur stehende Reaktoren sind realisierbar.
- Die Kühlfläche pro Katalysatorvolumen ist nur in engen Grenzen wählbar.

US-A-4,544,544 offenbart einen zylinderförmigen Reaktor, der durch parallele Platten und durch durchlässige Abschlüsse in Reaktionsräume aufgeteilt wird, die mit Katalysator gefüllt sind. Die parallelen Platten werden von einem Kühlmittel durchflossen. Die Anordnung der Reaktionsräume und der parallelen Wärmeaustauscherplatten ist derart, daß ein Gas zunächst in einen Reaktionsraum eintritt und nacheinander zwischen den Wärmeleitplatten hindurch in weitere, sich jeweils zwischen zwei Wärmeplatten befindende, Reaktionsräume geleitet wird.

Aus US-A-3,127,247 ist ein Reaktor bekannt, der einen vertikalen zylindrischen Tank umfaßt. In diesem zylindrischen Tank befinden sich mehrer konzentrische Hohlräume, welche von zylindrischen Platten begrenzt sind, die von Kühlmittel durchflossen werden können. Die ringförmigen Hohlräume werden mit Katalysator befüllt, wodurch kühlbare, als konzentrische Ringe angeordnete Reaktionsräume in dem Reaktor vorliegen. Die Wände dieser Hohlräume sind fest mit dem Boden und dem Deckel des Reaktors verbunden.

Aufgabe der Erfindung ist daher eine einfachere Konstruktion des Reaktors verbunden mit größerer Sicherheit im Betrieb bei Störungen und Vermeidung der genannten Nachteile.

Diese Aufgabe wird erfindungsgemäß gelöst von einem Reaktor mit den Merkmalen des Anspruchs 1. Ausführungsvarianten der Erfindung sind Gegenstand von Unteransprüchen.

Vorteilhaft bei der Erfindung ist, daß beim betreffenden Reaktor die gekühlten Trennwände mit Hilfe von Metallplatten/metallischen Baueinheiten gebildet sind und zur Kühlung in den Metallplatten/Baueinheiten Hohl-oder Zwischenräume in Form von Kanälen zur Aufnahme und zum Durchleiten eines Kühlmediums angeordnet sind.

Mit den durch die gekühlten Trennwände verwirklichten separaten Reaktionsräumen ist auch bei den genannten Betriebsstörungen, eine Überhitzung benachbarter Reaktionsräume ausgeschlossen. Erfindungsgemäße Metallplatten/metallischen Baueinheiten sind als kühlbare oder heizbare Panele im Handel erhältlich und ermöglichen kostengünstige Lösungen für die Reaktoreinbauten.

Bei dem erfindungsgemäßen Reaktor werden jeweils mehrere Metallplatten, vorzugsweise senkrecht, mit Abstand voneinander zu einem Metallplattenpaket zusammengefügt und bilden so einen Freiraum, in den die Katalysatorpartikel geschüttet werden. Rohrböden entfallen und die Reaktionsräume zwischen den Platten sind ähnlich zu befüllen wie ein Festbett ohne Kühlung. Dies ist eine wesentliche Verbesserung gegenüber einem Reaktor nach dem Stand der Technik.

Die Metallplattenpakete werden aus ebenen, vorzugsweise parallel angeordneten Platten gebildet. Solche Platten, auch Plattenpakete, sind kostengünstig im Handel erhältlich.

Bei dem erfindungsgemäßen Reaktor werden mehrere Metallplattenpakete so nebeneinander im Reaktorbehälter angeordnet, daß sie ein Modul aus Plattenpaketen bilden, in dem die Plattenpakete parallel vom Einsatzgas durchströmt werden. Dies ist, insbesondere auch in liegenden Behältern, leicht zu verwirklichen. Dadurch werden die Grenzen der Baubarkeit zu wesentlich größeren Einheiten verschoben und es werden bei einer Verwendung des erfindungsgemäßen Reaktors niedrige Druckabfälle ermöglicht.

Es können vorzugsweise auch mehrere Module entweder im gleichen Reaktorbehälter, vorzugsweise übereinander, oder in mehreren Reaktorbehältern angeordnet, parallel oder nacheinander vom Einsatzgas durchströmt werden. Zusammen mit der Wahlmöglichkeit zwischen stehenden und liegenden Reaktorbehältem kann der Reaktor so dem verfügbaren Platz und dem zulässigen Druckabfall im Reaktor optimal angepaßt werden.

Weitere Vorteile des erfindungsgemäßen Reaktors ergeben sich aus dem Zusammenwirken der Merkmale der Erfindung mit denen ihrer günstigen Ausführungsformen:
- Der Reaktormantel muß nur für den Druck des Reaktionsgases ausgelegt werden und beispielsweise nicht für den höheren Druck des Dampfes, der bei der Kühlung mit verdampfendem Kesselspeisewasser erzeugt wird.
- Der Reaktor benötigt keine Rohrböden und ist deshalb wesentlich leichter als ein Reaktor nach dem Stand der Technik. Außerdem können die Metallplatten aus Edelstahl gebaut werden, so daß die o.g. Probleme bei der Verwendung von C-Stahl entfallen.
- Die Kühlfläche pro Katalysatorvolumen ist in sehr weiten Grenzen frei wählbar.
- Weil die erfindungsgemäßen Reaktoren bei gleicher Leistung wesentlich leichter sind, werden Transport, Montage und Fundamente billiger als bei Reaktoren nach dem Stand der Technik.
- Baubarkeitsgrenzen und Sicherheitsbedenken begrenzen die an einem Standort installierbare Produktionskapazität nicht.

Bei einer vorteilhaften Verwendung des erfindungsgemäßen Reaktors wird zwischen den gekühlten Trennwänden des Reaktors eine exotherme katalytische Reaktion mit starker Wärmetönung durchgeführt.

Der Reaktor kann beispielsweise bei der Reaktion von Azetylen zu Ethylen verwendet werden. Bei dieser Verwendung ermöglicht es der erfindungsgemäße Reaktor, das Verfahren wie unten erläutert zu vereinfachen und zugleich den gekühlten Reaktor einfacher, sicherer und billiger zu machen und seine Baubarkeitsgrenzen wesentlich in Richtung größerer Einheiten zu verschieben.

Bei der Verwendung des erfindungsgemäßen Reaktors werden nämlich auf sehr einfache Art und Weise die Reaktionsräume im Reaktor von gekühlten Trennwänden begrenzt und die Kühlung durch ein innerhalb der Trennwände strömendes Fluid bewerkstelligt, indem erfindungsgemäß beim Reaktor die gekühlten Trennwände mit Metallplatten gebildet werden und in den Metallplatten Hohlräume in Form von Kanälen zur Aufnahme und zum Durchleiten mindestens eines Fluids angeordnet sind.

Durch die Trennwände werden separate Reaktionsräume für die Reaktion von Azetylen zu Ethylen gebildet. Sollte tatsächlich eine Überreaktion in einem dieser Reaktionsräume auftreten, bleibt die Überreaktion auf diesen kleinen Raum begrenzt und erfaßt nicht den gesamten Reaktor. Das erhöht ganz wesentlich die Sicherheit der Produktion und erst dadurch wird es akzeptabel, wesentlich größere Reaktoreinheiten als nach dem Stand der Technik möglich zu bauen.

Da Flächen aus Metallplatten billig herzustellen sind, kann man auch ohne großen finanziellen Aufwand zusätzliche Wärmeaustauschfläche zur Verfügung stellen. Insbesondere kann am Gasaustritt des Reaktors Inertmaterial statt Katalysator eingesetzt werden, so daß zwar keine Reaktion an dieser Stelle des Reaktors mehr stattfindet, wohl aber Kühlung durch die Metallplatten. So wird sichergestellt, daß kein heißes Gas aus einer Überreaktion den Produktgasstrom aufheizt und dort ebenfalls die Überreaktion auslösen kann.

Da die Platten wenig Bearbeitung brauchen, ist man in der Materialwahl wenig eingeschränkt. Insbesondere kann man Korrosionszuschläge leichter realisieren als bei Rohren. Besonders hochwertige und teure Materialien, wie z.B. Hastelloy, sind als Bleche am Markt leichter erhältlich als Rohre.

Weiterhin erlaubt es der niedrige Druckabfall im erfindungsgemäßen Reaktor, mit größerer Raumgeschwindigkeit das Katalysatorbett zu durchströmen. Dabei nimmt die Selektivität des Katalysators zu, so daß der Sicherheitsabstand zu einer unerwünschten Überreaktion größer wird.

Auf diese Verwendung ist der erfindungsgemäße Reaktor jedoch nicht beschränkt. Andere Verwendungen lassen sich aus den Eigenschaften des Reaktors in ähnlicher Weise ableiten. Insbesondere können andere Reaktionen mit starker Wärmetönung in diesem Reaktor durchgeführt werden, wie z.B. die Epoxidation von Olefinen, die CO-Konvertierung zur H₂-Gewinnung, die Direktoxidation von H₂S zu Elementarschwefel, die Clausreaktion, die Hydrierung von Kohlenwasserstoffen, insbesondere die selektive Hydrierung von Kohlenwasserstoffen wie die C₂H₂ -Hydrierung zu C₂H₄, die Oxidation von SO₂ zu SO₃, die Methanolsynthese, die Methansynthese, die Fischer-Tropsch-Synthese, und die NH₃ -Synthese. Alle diese Reaktionen sind exotherm. Prinzipiell ist der Reaktor jedoch ebenso geeignet, um darin endotherme Reaktionen durchzuführen. Ein spezielles Beispiel dieser Art ist die Verwendung des Reaktors in einer Clausanlage, die im letzten Reaktor unterhalb des Schwefeltaupunkts betrieben wird (=SubDewPoint, SDP-Betrieb). Wie in EP 0 283 793 beschrieben werden in diesem Verfahren zwei Reaktoren zyklisch vertauscht und dabei der schwefelbeladene Reaktor regeneriert. Zu Beginn dieser Regenerierung muß viel Wärme aufgebracht werden, um den auf dem Katalysator abgelagerten Schwefel zu verdampfen. Der erfindungsgemäße Reaktor kann diesen Vorgang unterstützen, indem ein Heizmedium, z.B. heißes Kesselspeisewasser zur Erzeugung von Mitteldruckdampf in die Metallplatten .geleitet wird, dadurch den Reaktor schnell erwärmt und so den Schwefel abtreibt. Im Normalbetrieb erwärmt sich dann der Reaktor weiter. Wenn die Temperatur des Mitteldruckdampfs erreicht ist, wendet sich die Aufgabe des Kesselspeisewassers von einer Heizung in ein Kühlmedium, d.h. durch Verdampfung wird dann Mitteldruckdampf erzeugt, der den Reaktor kühlt.

Die Erfindung wird anhand einer Ausführungsform mit einer Figur näher erläutert.

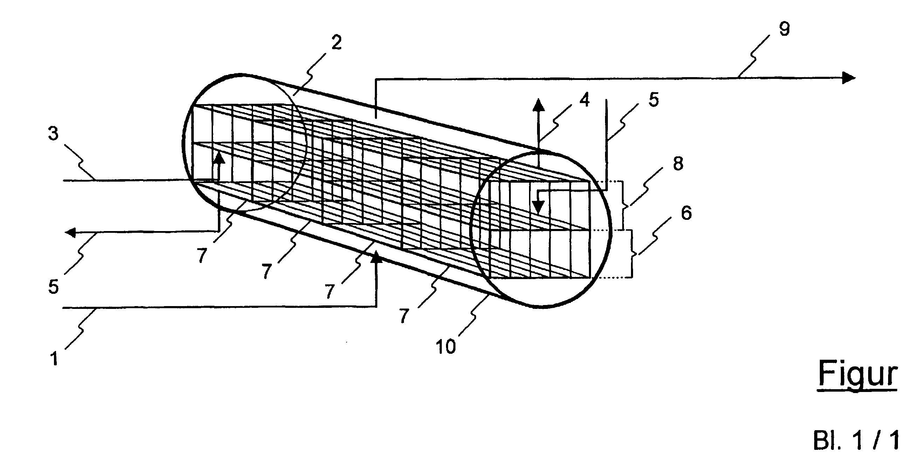

Die Figur zeigt schematisch eine Ausführung des erfindungsgemäßen Reaktors und seine Verwendung in einer Anlage zur selektiven Hydrierung von Azetylen zu Ethylen.

Die Figur zeigt einen Reaktor 2 in Form eines liegenden Behälters 10, in dem zwei Module 6, 8 aus Plattenpaketen 7 angeordnet sind. Angedeutet ist, daß die Pakete 7 aus gekühlten parallelen Platten bestehen, deren Kanten in der Figur als Linie dargestellt sind. In ihrem Aufbau können die gekühlten Platten den Heizkörpern für Wohnund Büroräume ähneln. Die Katalysatorschüttungen zwischen den Platten sind in der Figur nicht dargestellt.

Im Betrieb wird dem Reaktor 2 ein Einsatzgas über Zuleitung 1 zugeführt, wobei der Reaktor mit flüssigem Methanol 3 gekühlt sein kann, so daß dabei Methanol dampfförmig anfällt, wobei dieses über Ableitung 4 abfließt. Das eingesetzte Gas 1, z.B. Ethylen mit geringen Mengen Azetylen und mit Wasserstoff überstöchiometrisch verglichen mit einer zur Hydrierung des Azetylens zu Ethylen benötigten Menge, wird mit Hilfe eines Wärmeträgers 5, der wiederum Methanol sein kann, in einem Anwärmmodul 6 mit vier Plattenpaketen 7 auf eine Anspringtemperatur der katalytisch exothermen Umsetzung des Azetylens zu Ethylen vorgewärmt und in einem anschließenden Reaktionsmodul 8 das Azetylen im Einsatz hydriert.

Das bezüglich des Azetylens hydrierte Gas verläßt den Reaktionsmodul 8 des Reaktors 2 in Leitung 9 als Ethylenprodukt mit der gewünschten Reinheit oder das Ethlyen wir weiteren in der Figur nicht dargestellten Reinigungsschritten zugeführt. Der Anwärmmodul 6 und der Reaktionsmodul 8 sind in einem gemeinsamen Reaktorbehälter 10 angeordnet. Der Anwärmmodul 6 enthält Inertpartikel, der Reaktionsmodul 8 Katalysatorpartikel zwischen den Metallplatten.

Eine vorteilhafte Anwendung des erfindungsgemäßen Reaktors soll anhand eines Beispiels erläutert werden.

### Beispiel 1: Anwendung in Ethylenanlagen zur Azetylenhydrierung

Zur Hydrierung von C₂H₂ werden bisher Reaktoren eingesetzt, in denen Geradrohrtauscher mit Katalysator in den Rohren für die notwendige Kühlung sorgen. Solche Reaktoren für eine Ethylenanlage von ca. 600 000 Jahrestonnen Kapazität für die Ethylenerzeugung hat folgende charakteristischen Daten:
- Abmessungen:: 2 Reaktoren á 4,5 m Ø x 13,8 m, davon 1 Reaktor als 100% Reserve
- Gewicht:: je ca. 138 t
- Material:: Kohlenstoffstahl

Erfindungsgemäße Reaktoren für eine Anlage mit 600 000 Jahrestonnen Produktionsleistung weisen beispielsweise folgende Daten auf:
- Abmessungen:: 2 Reaktoren ä 3,8 m Ø x 16 m, davon 1 Reaktor als 100% Reserve
- Gewicht:: ca. 100 t, davon ca. 80 t Kohlenstoffstahl, ca 20 t Edelstahl
- Material:: Mantel: Kohlenstoffstahl, Platten: Edelstahl

Der Trend zu immer größeren Ethylenanlagen hält an. Angestrebt werden Produktionsmengen von 1 000 000 Jahrestonnen pro Strang. Beim konventionellen Reaktor zur C₂H₂ -Hydrierung ist jedoch die Baubarkeitsgrenze bei ca. 600 000 Jahrestonnen Produktionkapazität erreicht. Wenn man an dieser Stelle zu Mehrsträngigkeit übergehen muß, entstehen erhebliche Zusatzkosten, weil neben den Parallelreaktoren selbst auch noch Rohrleitungen, Regelgeräte und Steuerungen für gleichmäßige Beaufschlagung aller Reaktoren erforderlich sind. Im Plattenaustauscher-Reaktor wird das verfügbare Volumen viel effizienter genutzt, so daß bei gleichen Außenabmessungen in einem Plattenaustauscher- Reaktor ca. 40% mehr Katalysatorvolumen gekühlt unterzubringen ist, als in einem konventionellen Reaktor. Damit ist selbst ohne die prinzipiell mögliche Baustellenfertigung bei Plattenaustauscher-Reaktoren ca. 40% mehr Durchsatz zu realisieren, also statt 600 000 Jahrestonnen Ethylen ca. 840 000 Jahrestonnen. Bei Baustellenfertigung ist auch eine noch größere Ethylenerzeugung mit nur einem Reaktor für die C₂H₂ -Hydrierung durchführbar.

Ein solcher herkömmlicher Reaktor erfordert Material von ca. 140 t Gewicht. Die schwersten Einzelteile sind dabei die Rohrböden, die je über 20 t wiegen. Diese Rohrböden fallen beim erfindungsgemäßen Reaktor weg, so daß allein dadurch bereits Material und Gewicht eingespart wird. Ein erfindungsgemäßer Reaktor für 600 000 Jahrestonnen Ethylenerzeugung wiegt nur ca. 80 t. Darüber hinaus entstehen weitere Vorteile durch einen geringeren Druckabfall im Gasweg und eine kürzere Stillstandszeit beim Austausch des Katalysators.

## Patentansprüche

1. Reaktor zur Durchführung von Reaktionen mit starker Wärmetönung mit Katalysatorpartikeln zwischen gekühlten Trennwänden in mindestens einem Reaktorbehälter, wobei die gekühlten Trennwände mit Hilfe von Metallplatten/metallischen Baueinheiten gebildet sind und zur Kühlung in den Metallplatten/metallischen Baueinheiten Hohl- oder Zwischenräume in Form von Kanälen zur Aufnahme und zum Durchleiten eines Kühlmediums angeordnet sind, wobei jeweils mehrere Metallplatten mit Abstand voneinander und vorzugsweise senkrecht zu einem Metallplattenpaket zusammengefügt sind und so einen Freiraum/Freiräume bilden, in den/die die Katalysatorpartikel geschüttet werden und wobei die Metallplattenpakete aus ebenen, vorzugsweise parallel angeordneten Platten gebildet sind, **dadurch gekennzeichnet, daß** mehrere Metallplattenpakete so nebeneinander im Reaktorbehälter angeordnet werden, daß sie ein Modul aus Plattenpaketen bilden, in dem die Plattenpakete parallel vom Einsatzgas durchströmt werden.

2. Reaktor nach Anspruch 1, **dadurch gekennzeichnet, daß** mehrere Module entweder im gleichen Reaktorbehälter, vorzugsweise übereinander, oder in mehreren Reaktorbehältern angeordnet nacheinander vom Einsatzgas durchströmt werden.

3. Verwendung des Reaktors nach einem der Ansprüche 1 oder 2, **dadurch gekennzeichnet, daß** zwischen den gekühlten Trennwänden des Reaktors eine katalytische Reaktion mit starker Wärmetönung durchgeführt wird.

4. Verwendung nach Anspruch 3 bei der CO-Konvertierung.

5. Verwendung nach Anspruch 3 bei der Epoxidation von Olefinen zur Olefinoxidherstellung.

6. Verwendung nach Anspruch 3 bei der selektiven Hydrierung von Kohlenwasserstoffen, beispielsweise von C₂H₂ zu C₂H₄.

7. Verwendung nach Anspruch 3 bei der nicht-selektiven Hydrierung von Kohlenwasserstoffen, beispielsweise von C₂H₄ zu C₂H₆.

8. Verwendung nach Anspruch 3 bei der Methansynthese.

9. Verwendung nach Anspruch 3 bei der Methanolsynthese.

10. Verwendung nach Anspruch 3 bei der Clausreaktion.

11. Verwendung nach Anspruch 3 bei der Direktoxidation von H₂S zu Elementarschwefel.

12. Verwendung nach Anspruch 3 bei der Fischer-Tropsch-Synthese.

13. Verwendung nach Anspruch 3 bei der Oxidation von SO₂ zu SO₃.

14. Verwendung nach Anspruch 3 bei der Synthese von NH₃.

## Claims

1. Reactor for the execution of reactions with high enthalpy change with catalyst particles between cooled plates in at least one reactor vessel, in which the cooled plates are formed by metal plates/metallic units, in which are arranged for the cooling in the metal plates/metallic units hollow channels or spaces in the form of channels for the reception and for the conveying of a cooling medium, whereby several metal plates with a fixed distance from one another are combined to form a metal plate package which forms a free space into which catalyst particles are poured, and where the metal plate packages are formed out of even plates arranged preferably in parallel, **characterized by** arranging several metal plate packages so next to one another in the reactor vessel. that they form a module out of plate packages, through which plate packages the feed gas flows in parallel.

2. Reactor according to claim 1, **characterized by** arranging several modules either in the same reactor vessel, preferably one over the other, or in several reactor vessels arranged so that the feed gas flows through them in series each.

3. Use of the reactor according to claims 1 or 2, **characterized by** a catalytic reaction with high enthalpy change being carried out between the cooled plates

4. Use according to claim 3 for the CO shift.

5. Use according to claim 3 in the epoxidation of olefines for the production of olefine oxide.

6. Use according to claim 3 in the selective hydrogenation of hydrocarbons. for example from C2H2 to C2H4.

7. Use according to claim 3 in the not selective hydrogenation of hydrocarbons, for example from C2H4 to C2H6.

8. Use according to claim 3 in the methane synthesis.

9. Use according to claim 3 in the methanol synthesis.

10. Use according to claim 3 in the Claus reaction.

11. Use according to claim, 3 in the direct oxidation of H2S to elemental sulfur.

12. Use according to claim 3 in the Fischer- Tropsch synthesis.

13. Use according to claim 3 in the oxidation of SO2 to SO3.

14. Use according to claim 3 in the synthesis of NH3.

## Revendications

1. Réacteur pour effectuer des réactions ayant une forte chaleur de réaction avec des particules de catalyseur entre des parois séparatrices refroidies dans au moins un récipient réacteur, dans lequel les parois séparatrices refroidies sont formées au moyen de plaques de métal/ ensembles métalliques et, pour le refroidissement, des creux ou des interstices sous la forme de canaux sont agencés dans les plaques de métal/ensembles métalliques afin de recevoir et véhiculer un milieu réfrigérant, dans lequel plusieurs plaques de métal sont respectivement assemblées avec un espacement les unes par rapport aux autres et de préférence perpendiculairement à un empilage de plaques de métal et forment ainsi un ou des espaces libres dans le ou lesquels les particules de catalyseur sont déversées et dans lequel les empilages de plaques de métal sont formés par des plaques planes, de préférence disposées parallèlement, **caractérisé en ce que** plusieurs empilages de plaques de métal sont disposés les uns à côté des autres dans le récipient réacteur de façon à former un module d'empilages de plaques dans lequel les empilages de plaques sont parcourus parallèlement par le flux de gaz introduit.

2. Réacteur selon la revendication 1, **caractérisé en ce que** plusieurs modules, disposés soit dans le même récipient réacteur, de préférence les uns au-dessus des autres, soit dans plusieurs récipients réacteurs à la suite les uns des autres, sont parcourus par le flux de gaz introduit.

3. Utilisation du réacteur selon la revendication 1 ou la revendication 2, **caractérisée en ce qu'**une réaction catalytique ayant une forte chaleur de réaction est effectuée entre les parois séparatrices refroidies du réacteur.

4. Utilisation selon la revendication 3 dans la conversion de CO.

5. Utilisation selon la revendication 3 dans l'époxydation d'oléfines pour la production d'oxydes d'oléfines.

6. Utilisation selon la revendication 3 dans l'hydrogénation sélective d'hydrocarbures, par exemple de C₂H₂ en C₂H₄.

7. Utilisation selon la revendication 3 dans l'hydrogénation non sélective d'hydrocarbures, par exemple de C₂H₄ en C₂H₅.

8. Utilisation selon la revendication 3 dans la synthèse de méthane.

9. Utilisation selon la revendication 3 dans la synthèse de méthanol.

10. Utilisation selon la revendication 3 dans la réaction de Claus.

11. Utilisation selon la revendication 3 dans l'oxydation directe de H₂S en soufre élémentaire.

12. Utilisation selon la revendication 3 dans la synthèse de Fischer-Tropsch.

13. Utilisation selon la revendication 3 dans l'oxydation de SO₂ en SO₃.

14. Utilisation selon la revendication 3 dans la synthèse de NH₃.
